(19) Europäisches Patentamt
European Patent Office
Office européen des brevets

(11) Numéro de publication: **0 507 696 B1**

# (12) FASCICULE DE BREVET EUROPEEN

(45) Date de publication de fascicule du brevet: **09.08.95**

(51) Int. Cl.[6]: **C07D 403/12**, A61K 31/415, A61K 31/40, A61K 31/55, A61K 31/41

(21) Numéro de dépôt: **92400940.0**

(22) Date de dépôt: **03.04.92**

## (54) Antihistaminiques non sédatifs, dérivés de benzimidazole, leur procédé de préparation et leur utilisation en tant que médicaments.

(30) Priorité: **05.04.91 FR 9104171**

(43) Date de publication de la demande:
**07.10.92 Bulletin 92/41**

(45) Mention de la délivrance du brevet:
**09.08.95 Bulletin 95/32**

(84) Etats contractants désignés:
**AT BE CH DE DK FR GB GR IT LI LU MC NL PT SE**

(56) Documents cités:
**EP-A- 0 139 993**
**US-A- 4 200 641**

(73) Titulaire: **LABORATORIOS DEL DR. ESTEVE, S.A.**
**Av. Mare de Deu de Montserrat, 221**
**E-08026 Barcelona (ES)**

(72) Inventeur: **Cuberes-Altisent, Maria Rosa**
**Rue Barcelona No 2-D,**
**Sant Cugat del Valles**
**Barcelone (ES)**
Inventeur: **Frigola Constansa, Jordi**
**Av. Diagonal, 299 at.1a**
**E-08013 Barcelone (ES)**
Inventeur: **Pares Corominas, Juan**
**Padilla, 349, 3o, 3a**
**E-08025 Barcelone (ES)**

(74) Mandataire: **Ahner, Francis et al**
**CABINET REGIMBEAU**
**26, avenue Kléber**
**F-75116 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention se rapporte à de nouveaux dérivés de benzimidazole, leur procédé de préparation ainsi qu'à leur application en tant que médicaments.

Les composés objet de la présente invention répondent à la formule générale I

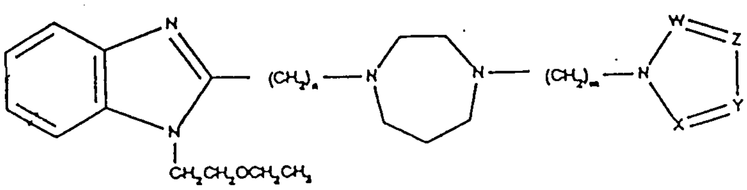

I

dans laquelle :

- n peut avoir les valeurs 0 ou 1,
- m peut avoir les valeurs 2 à 4,
- X, Y, Z et W, égaux ou différents, représentent un atome d'azote ou un atome de carbone lié à un atome d'hydrogène, à un halogène ou à un radical alkyle, carboxyle ou alkyloxycarbonyle.

Dans la littérature scientifique on connait déjà des dérivés de benzimidazole avec différentes activitées biologiques, comme par exemple, activité analgésique et antiinflammatoire (Japan Kokai 75, 126, 682), activité antisécrétoire gastrique (EP 246.126 et EP 5129); activité antihistaminique (J. Jilek et al., Collect. Czech. Chem. Commun. 1988, 53, 870-83; US Patent 4.200.641; Drugs of the Future, VII; 10-1, 1982; R. Iemura et al., J. Med. Chem., 1986, 29, 1178-1183; R. Iemura et al., J. Heteroxycyclic. Chem., 1987, 24, 31-37; Demande de brevet français FR 2 665 161. Les composés objet de la présente invention sont des nouveaux dérivés de benzimidazole, en concret 1-(2-étoxyéthyl)-2-{ω[ω(azol-1-yl)alkyl]hexahydro-1,4-diazépin-1-yl alkyl}benzimidazole, lesquels seront nommés, dans cette invention, comme 1-(2-étoxyéthyl)-2-{ω[ω-(azol-1-yl)alkyl] homopipérazin-1-yl alkyl}benzimidazole. Nous avons découvert que ces nouveaux derivés présentent une très bonne activité antihistaminique et ils n'ont pas d'effets secondaires sur le système nerveux central.

Les nouveaux dérivés de formule générale I peuvent être preparés, conformément à l'invention, selon l'une quelconque des méthodes suivantes:

Méthode A .- Par réaction d'un composé de formule générale IIa

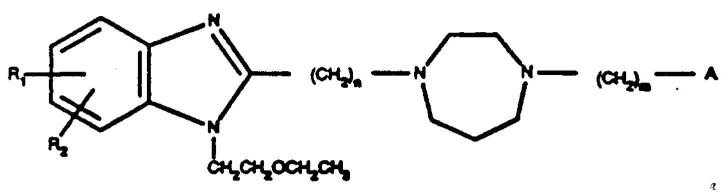

IIa

ou bien IIb

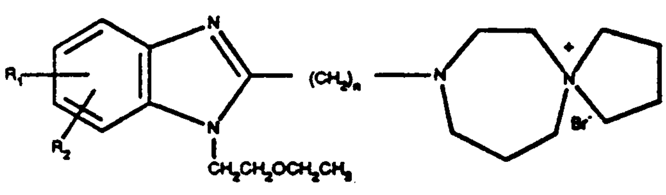

IIb

dans lesquelles $R_1$, $R_2$ représentent chacun un atome d'hydrogène, et n et m ont les significations mentionnées précédemment, et A représente un atome d'halogène, ou un bon "groupe partant" choisi parmi le tosyloxy ou le mésyloxy, avec un composé de formule générale III

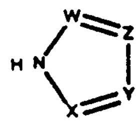

III

dans laquelle X, Y, Z et W ont les significations mentionnées précédemment.

La réaction s'effectue en présence d'un solvant adéquat, par exemple le diméthylsulfoxyde, la diméthylformamide, des alcools, des hydrocarbures, aromatiques ou non, des éthers, tels le dioxanne ou l'éther diphénylique, ou des mélanges de ces solvants. Cette réaction est avantageusement conduite en présence d'une base telle les hydroxydes, les carbonates ou les bicarbonates des métaux alcalins, ou bien d'un mélange de ces bases. On peut employer aussi les hydrures des métaux alcalins. Les températures les plus adéquates oscillent entre la température ambiante et la température de reflux du solvant, et le temps réactionnel est compris entre 1 heure et 24 heures.

Méthode B.- Par réaction d'un composé de formule générale IIa, dans laquelle A représente un radical $-NH_2$,avec le 2,5-diméthoxytétrahydrofurane.

La réaction s'effectue en présence d'un solvant adéquat, par exemple l'acide acétique, l'eau, des alcools, des cétones ou des mélanges de ces solvants. Les températures les plus adéquates oscillent entre la température ambiante et la température de reflux du solvant, et le temps réactionnel est compris entre quelques minutes et 24 heures.

Méthode C.- Par réaction d'un composé de formule générale IV

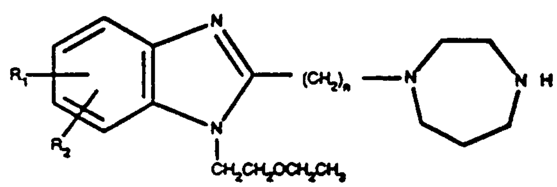

IV

dans laquelle $R_1$, $R_2$ et n ont les significations mentionnées précédemment, avec un composé de formule générale V

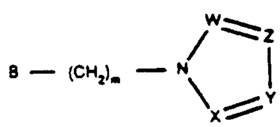

V

où X, Y, Z, W et m ont les significations mentionnées précédemment, et B représente un atome d'halogène, ou un bon "groupe partant" choisi parmi le tosyloxy ou le mésyloxy.

La réaction s'effectue en présence d'un solvant adéquat, par exemple le diméthylsulfoxyde, la diméthylformamide, des alcools, des hydrocarbures, aromatiques ou non, des éthers, tels le dioxanne ou l'éther diphénylique, ou des mélanges de ces solvants. Cette réaction est avantageusement conduite en présence d'une base telle les hydroxydes, les carbonates ou les bicarbonates des métaux alcalins, ou bien d'un mélange de ces bases. Les températures les plus adéquates oscillent entre la température ambiante et la température de reflux du solvant, et le temps réactionnel est compris entre 1 heure et 24 heures.

Dans les exemples suivants on indique la préparation des nouveaux dérivés selon l'invention. Les exemples ci-aprés, donnés à simple titre d'illustration, ne doivent cependant, en aucune façon, limiter l'étendue de l'invention.

Méthode A

Exemple 1.- Préparation de 1-(2-éthoxyéthyl)-2-{4-[4--(pyrazol-1-yl)butyl]homopipérazin-1-yl-methyl}benzimidazole.

a) 1-(2-éthoxyéthyl)-2-(4-benzyl-1H-homopipérazin-1-yl-méthyl)benzimidazole.

A une suspension de 2,04 g (46,7 mmoles) de NaH (55% en huile minérale) on ajoute, lentement, une solution de 13,6 g (42,5 mmoles) de 1H-2-(4-benzyl-1H-homopipérazin-1-yl-méthyl)benzimidazole en 20 ml de diméthylformamide (DMF). On chauffe à 60-70 °C pendant 1 heure, et puis on ajoute une solution de 5,1 g (46,7 mmoles) de 1-chloro-2-étoxyéthane en 5 ml DMF.

On maintient, sous agitation, aux mêmes conditions pendant 5 heures. On verse sur l'eau et on extrait avec de l'acétate d'éthyle, on lave avec de l'eau, on sèche la phase organique avec $Na_2SO_4$, on filtre et on évapore. L'huile résultant est purifié sur une colonne chromatographique de sillice. En éluant avec chloroforme-méthanol 99:1 on obtient 5,65 g (50%) de 1-(2-éthoxyéthyl)-2-(4-benzyl-1H-homopipérazin-1-yl-méthyl)benzimidazole, et avec chloroforme-méthanol 97:3 on récupère 4,3 g (32%) de produit de départ qui n'a pas réagi.
$^1$H-RMN ($CDCl_3$): $\delta$ 1,12 (t,3H); 1,79 (m,2H); 2,69 (m,8H); 3,41 (q,2H); 3,63 (s,2H); 3,76 (t,2H); 3,98 (s,2H); 4,55 (t,2H); 7,25 (m,8H); 7,7 (m,1H).

b) 1-(2-éthoxyéthyl)-2-(homopipérazin-1-yl-méthyl)benzimidazole.

On chauffe à 60 °C une solution de 5,94 g (15,15 mmoles) de 1-(2-éthoxyéthyl)-2-(4-benzyl-1H-homopipérazin-1-yl-méthyl)benzimidazole, en 80 ml d'acide acétique 80%, avec 4,02 g de Pd/C 5% (contenu en eau: 50%) sous une atmosphère d'hydrogène à 5 atm., pendant 16 heures. On filtre et on évapore à sec. On reprend le résidu avec du chloroforme et on lave avec NaOH 20%, avec de l'eau, et on sèche avec $Na_2SO_4$, on filtre et on évapore. On obtient 3,65 g (80%) de 1-(2-éthoxyéthyl)-2-(homopipérazin-1-yl-méthyl)benzimidazole.
$^1$H-RMN ($CDCl_3$): $\delta$ 1,12 (t,3H); 1,78 (m,2H); 2,28 (s élargie,1H); 2,74-3,05 (m,8H); 3,41 (q,2H); 3,76 (t,2H); 4,02 (s,2H); 4,56 (t,2H); 7,25 (m,3H); 7,7 (m,1H).
IR(film): 3312, 1463, 1119, 744 $cm^{-1}$

c) Bromure de 1-(2-éthoxyéthyl)-2-(8-méthylaza-5-azoniaspiro [4,6]undécane)benzimidazole.

On met à reflux pendant 16 heures un mélange de 4 g (13,24 mmoles) de 1-(2-éthoxyéthyl)-2-(homopipérazin-1-yl-méthyl)benzimidazole, 3,29 g (15,23 mmoles) de 1,4-dibromobutane et 2,5 g (18,1 mmoles) de carbonate de potassium dans 40 ml de chloroforme. On refroidit, on filtre et on évapore. On triture le résidu dans l'éther éthylique et on obtient 5,6 g (97%) d'un solide hygroscopique qu'on utilise tel quel, sans autre purification.
$^1$H-RMN ($CDCl_3$): $\delta$ 1,06 (t,3H); 2,24 (m,6H); 2,96-3,51 (m,8H); 3,72-3,90 (m,8H); 4,15 (s,2H); 4,53 (t,2); 7,30 (m,3H); 7,74 (m,1H).

d) 1-(2-éthoxyéthyl)-2-{4-[4-(pyrazol-1-yl)butyl]homopipérazin-1-yl-méthyl}benzimidazole.

On chauffe à reflux, pendant 16 heures, un mélange de 3 g (6,86 mmoles) de bromure de 1-(2-éthoxyéthyl)-2-(8-méthylaza-5-azoniaspiro[4,6]undécane)benzimidazole, 0,56 g (8,24 mmoles) de pyrazole, 1,8 g (13 mmoles) de carbonate de potassium et 30 ml de diméthylformamide. On refroidit, on filtre et on évapore le filtrat à sec. On reprend le résidu avec du chloroforme et on lave avec de l'eau. On sèche la phase organique avec $Na_2SO_4$, on filtre et on évapore. L'huile résultant est purifié sur une colonne chromatographique de sillice (éluant: chloroforme-méthanol 9:1). On obtient ainsi 1,40 g (48%) de 1-(2-éthoxyéthyl)-2-{4-[4-(pyrazol-1-yl)butyl]homopipérazin-1-yl-méthyl}benzimidazole, sous forme de huile.

Les données spectroscopiques pour son identification sont exposées dans les Tableaux 1 et 2.

Exemple 3.- Préparation de 1-(2-éthoxyéthyl)-2-{4-[4-(4,5-dichloro-2-méthylimidazol-1-yl)butyl]-homopipérazin-1-yl-méthyl}benzimidazole.

La préparation s'effectue de manière tout à fait semblable à celle exposée à l'exemple 1, avec un rendement de 36%.

Les données spectroscopiques pour son identification sont exposées dans les Tableaux 1 et 2.

Exemple 5.- Préparation de 1-(2-éthoxyéthyl)-2-{4-[4-(4-carboxypyrazol-1-yl)butyl]homopipérazin-1-yl}benzimidazole

a) Bromure de 1-(2-éthoxyéthyl)-2-(8-aza-5-azoniaspiro[4,6]undécane)benzimidazole.

La préparation s'effectue avec la même procédure que celle exposée à l'exemple 1 c, avec un rendement de 97%.
$^1$H-RMN ($CDCl_3$): δ 1,09 (t,3H); 1,9-2,4 (m,6H); 3,42 (q,2H); 3,82 (t,2H); 3,9-4,1 (m,12H); 4,26 (t,2H); 7,20 (m,3H); 7,50 (m,1H).

b) 1-(2-éthoxyéthyl)-2-{4-[4-(4-éthyloxycarbonylpyrazol-1-yl)butyl]homopipérazin-1-yl}benzimidazole.

La préparation s'effectue avec la même procédure que celle exposée à l'exemple 1 d, et on obtient le produit cru, qui est purifié sur une colonne chromatographigue de sillice (éluant: chloroforme-méthanol 95:5). Rendement 35%.
$^1$H-RMN ($CDCl_3$): δ 1,13 (t,3H); 1,33 (t,3H); 1,93 (m,6H); 2,6 (t,2H); 2,8 (m,4H); 3,35-3,82 (m,8H); 4,07-4,4 (m,6H); 7,1-7,25 (m,3H); 7,5 (m,1H); 7,85 (s,2H).

L'ester précédemment préparé est hydrolysé par traitement avec de la soude à 10% pendant 15 heures à température ambiante, d'une solution dans l'éthanol. On évapore l'alcool et la solution aqueuse est neutralisée avec de l'acide chlorhydrique. On évapore à sec et l'acide est extrait du residu par digestion avec isopropanol. Rendement 87%. Point de fusion >300 °C.

Les données spectroscopiques pour son identification sont exposées dans les Tableaux 1 et 2.

Méthode B

Exemple 2.- Préparation de 1-(2-éthoxyéthyl)-2-{4-[4-(pyrrol-1-yl)butyl]homopipérazin-1-yl-méthyl}benzimidazole.

On chauffe à reflux, pendant 25 minutes, une solution de 2,98 g (8 mmoles) de 1-(2-éthoxyéthyl)-2-{4-[4-aminobutyl)homopipérazin-1-yl-méthyl}benzimidazole et 1,06 g (8 mmoles) de 2,5-dimétoxytétrahydrofurane dans 30 ml d'acide acétique. On refoidit, on verse sur l'eau glacée, on neutralise avec $NaHCO_3$ et on extrait avec du chloroforme. On sèche avec $Na_2SO_4$ et on évapore sous vide à sec. On obtient ainsi 3,2 g du composé cru qu'on purifie sur une colonne chromatographique de sillice (éluant: chloroforme-méthanol 92:8). Rendement 51%.

Les données spectroscopiques du composé sont les mêmes exposées dans l'exemple 2 de la méthode C.

Méthode C

Exemple 2.- Préparation de 1-(2-éthoxyéthyl)-2-{4-[4-(pyrrol-1-yl)butyl]homopipérazin-1-yl-méthyl}benzimidazole.

On met à reflux pendant 16 heures un mélange de 2,42 g (8 mmoles) de 1-(2-éthoxyéthyl)-2-(homopipérazin-1-yl-méthyl)benzimidazole, 1,39 g (8,8 mmoles) de 1-(4-chlorobutyl)pyrrole, 1,65 g (12 mmoles) de carbonate de potassium et 1,65 g (11 mmoles) de iodure de sodium dans 40 ml de méthyl éthyl cétone. On refroidit, on filtre et on évapore le filtrat à sec. On réprend le résidu avec du chloroforme et on lave avec de l'eau, on sèche, on filtre et on évapore sous vide. Le cru resultant est purifié sur une colonne chromatographique de sillice (éluant: chloroforme-méthanol 92:8) et on obtient 1,9 g (56%) de 1-(2-éthoxyéthyl)-2-{4-[4-(pyrrol-1-yl)butyl]homopipérazin-1-yl-méthyl}benzimidazole.

Les données spectroscopiques pour son identification sont exposées dans les Tableaux 1 et 2.

Exemple 4.- Préparation de 1-(2-éthoxyéthyl)-2-{4-[4-(pyrazol-1-yl)butyl]homopipérazin-1-yl}benzimidazole.

La préparation s'effectue de manière tout à fait semblable à celle exposée dans l'exemple précédent, et on obtient le composé, avec 49% de rendement, dont le sel avec l'acide maleique présente un point de fusion de 102-105 °C.

TABLEAU I

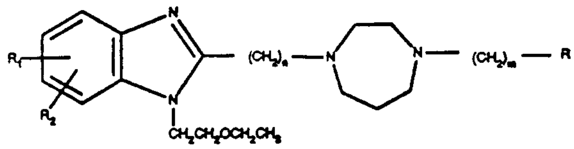

| Exemple nº | $R_1$ | $R_2$ | n | m | R | Méthode | IR ($cm^{-1}$) (film) |
|---|---|---|---|---|---|---|---|
| 1 | H | H | 1 | 4 | 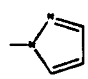 | A<br>C | 2938, 2869, 1464, 1119, 748, 619 |
| 2 | H | H | 1 | 4 | 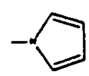 | B<br>C | 2936, 2870, 1463, 1120, 745, 725 |
| 3 | H | H | 1 | 4 | 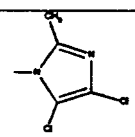 | A | 2937, 1464, 1408, 1246, 1120, 746 |
| 4 | H | H | 0 | 4 | 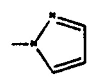 | C | maleate (KBr):<br>3000, 2890, 1619, 1579, 1470, 1358 |
| 5 | H | H | 0 | 4 | 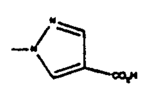 | A | 3600-3150, 1571, 1432, 670 |
| 6 | H | H | 0 | 4 | 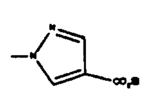 | A | 1715, 1565, 1465, 1120, 1040, 750 |

TABLEAU 2

| Exemple nº | $^1$H-RMN ($CDCl_3$) δ |
|---|---|
| 1 | 1,11 (t,3H); 1,41 (m,2H); 1,79 (m,4H); 2,37-2,74 (m,10H); 3,39 (q,2H); 3,75 (t,2H); 3,96 (s,2H); 4,12 (t,2H); 4,55 (t,2H); 6,22 (s élargie,1H); 7,2-7,47 (m,5H); 7,68 (m,1H) |
| 2 | 1,11 (t,3H); 1,47 (m,2H); 1,82 (m,4H); 2,48 (t,2H); 2,73 (m,8H); 3,39 (q,2H); 3,75 (t,2H); 3,87 (t,2H); 3,97(s,2H); 4,53(t,2H); 6,11 (m,2H); 6,63 (m,2H); 7,25 (m,3H); 7,67 (m,1H) |
| 3 | 1,11 (t,3H); 1,5-1,9 (m,6H); 2,36 (s,3H); 2,5-2,9 (m,10H); 3,39 (q,2H); 3,7-3,9 (dt,4H); 3,99 (s,2H); 4,54 (t,2H); 7,26 (m,3H); 7,68 (m,1H) |
| 4 | 1,13 (t,3H); 1,46 (m,2H); 1,95 (m,4H); 2,52 (t,2H); 2,79 (m,4H); 3,34-3,81 (m,8H); 4,05-4,19 (2t,4H); 6,20 (m,1H); 7,0-7,5 (m,6H) |
| 5 | $D_2O$: 0,93 (t,3H); 1,3-2,0 (m,6H); 2,6 (m,2H); 2,95 (m,4H); 3,17-3,62 (m,8H); 4,11 (m,4H); 7,1 (m,3H); 7,4 (m,1H); 7,87 (s,1H); 7,97 (s,1H) |
| 6 | 1,13 (t,3H); 1,33 (t,3H); 1,93 (m,6H); 2,6 (t,2H); 2,8 (m,4H); 3,35-3,82 (m,8H); 4,07-4,4 (m,6H); 7,1-7,25 (m,3H); 7,5 (m,1H); 7,85 (s,2H) (m,3H) |

Activité pharmacologique

Les produits objet de la présente invention sont des puissants antihistaminiques qui se caractérisent par le fait d'être exempts d'effets sédatifs, contrairement à la plupart des antihistaminiques connus.

Activité antihistaminique "in vivo"

L'activité antihistaminique a été étudiée en déterminant la protection face à la mortalité induite par le produit 48/80 chez le rat. Cet essai a été réalisé en suivant la technique décrite par C.J.E. Niemegeers et cols. (Arch. int. Pharmacodyn., 234, 164-176 (1978). Les produits objet de la présente invention s'administrent par voie i.p. aux rats. Après 60 minutes on administre le composé 48/80 (0,5 mg/kg, i.v.). L'activité protectrice se définit comme la survie des rats 4 heures après l'injection i.v. du 48/80.

On étudie l'activité des produits à plusieurs doses à fin de déterminer la dose capable de protéger le 50% des animaux (DE-50).

Ensuite on indique l'activité antihistaminique du produit de l'exemple 1. On compare cette activité avec celle de la difenhidramine, un antihistaminique de référence.

Activité antihistaminique "in vivo":

Protection de la mort induite par le 48/80

| Exemple nº | DE-50 (mg/kg, i.p.) |
|---|---|
| 1 | 0.04 |
| Difenhidramine | 5.4 |

Effet sédatif: 1) Test de Irwin

Pour étudier l'absence d'effet sédatif des produits objet de la présente invention, on les a administré aux rats par voie i.p. et on a observé le comportement des animaux, en suivant les normes décrites dans le test de S Irwin (Science, 136, 123-128 (1962)).

On montre ci-après le résultat obtenu pour le produit de l'exemple 1 dans les deux évaluations qui reflètent l'effet sédatif:

- Pas.: Passivité, sédation, prostration . Evaluation quantitative entre 0 et 3. On les réalise 1, 2 et 3 heures après le traitement.
- Atax.: Ataxie, on évalue les altérations de coordination dans la locomotion. On les évalue entre 0 et 3. On les réalise 1, 2 et 3 heures après le traitement.

Ci-après on résume les résultats de l'étude de l'effet sédatif du produit de l'exemple 1 de la présente invention, à titre d'exemple. Cette activité a été comparée avec celle de la difenhidramine, antihistaminique de référence. Ce produit montre un très faible effet sédatif, contrairement à la difenhidramine qui s'est avérée toxique à la dose de 80 mg/kg, i.p., à cause des effets dépresseurs du SNC.

**Effet sédatif: 1) Test de Irwin**

| Exemple nº | Dose (mg/kg) | Effet Pas. | Effet Atax. |
|---|---|---|---|
| 1 | (80) | 0 | 0.2 |
| Difenhidramine | (40) | 0 | 0.9 |
| | (80) | Toxique | |

Effet sédatif: 2) Potentiation du temps de sommeil induit par le pentobarbital

L'étude de la potentiation du temps de sommeil dû au pentobarbital a été réalisée en suivant la méthode décrite par L.E. Allen et cols. (Arz. Forsch. 24, (6), (1974)). Les produits étudiés ont été administrés par voie orale. Une heure plus tard on administre le pentobarbital de sodium (35 mg/kg, s.c.) et on détermine le temps que les animaux tardent à se réveiller. On compare le temps de sommeil avec un groupe d'animaux témoins, traités uniquement avec du pentobarbital de sodium.

A fin de compléter les études qui démontrent l'absence d'effet sédatif des produits objet de la présente invention on a comparé dans ce test l'activité d'un des produits (exemple 1) avec l'antihistaminique de référence, la difenhidramine. On présente ci-après les résultats de cet essai avec l'exemple 1 et la difenhidramine. Il est évident que la difenhidramine potentialise de façon significative le temps de sommeil à la dose de 20 mg/kg, alors que l'exemple 1 ne potentialise le temps de sommeil induit par le pentobarbital pas même à 160 mg/kg, dose maximale essayée.

Effet sédatif: 2) Potentialisation du temps de sommeil induit par le pentobarbital

| Exemple nº | Dose (mg/kg, p.o.) | Potentiation temps de sommeil | |
|---|---|---|---|
| 1 | 80 | 8 % | N.S. |
| | 160 | 1 % | N.S. |
| Difenhidramine | 10 | 22 % | N.S. |
| | 20 | 38 % | * |

N.S.: Non significatif

* : Différence significative avec le groupe témoin ($p < 0,05$)

On indiquera ci-après, à titre d'exemple, une forme galénique particulière des dérivés objet de la présente invention.

Comprimés

Formule par comprimé

| | |
|---|---|
| Composé de l'exemple 1 | 10,00 mg |
| Lactose | 54,00 mg |
| Amidon de maïs | 26,60 mg |
| Cellulose microcristalline | 18,00 mg |
| Polyvinylpyrrolidone | 6,00 mg |
| Croscarmellose de sodium | 3,60 mg |
| Dioxyde sillicique colloïdal | 0,60 mg |
| Stéarate de magnésium | 1,20 mg |
| | 120,00 mg |

### Revendications

1. Dérivés de benzimidazole caractérisés en ce qu'ils répondent à la formule générale I, et leurs sels thérapeutiquement acceptables,

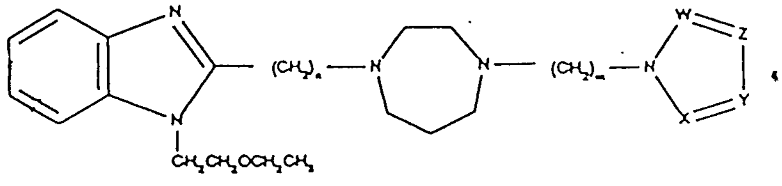

I

dans laquelle :

- n peut avoir les valeurs 0 ou 1,
- m peut avoir les valeurs 2 à 4,
- X, Y, Z et W, égaux ou différents, représentent un atome d'azote ou un atome de carbone lié à un atome d'hydrogène, à un halogène ou à un radical alkyle, carboxyle ou alkyloxycarbonyle.

**2.** Les composés répondant à la formule générale I selon la revendication 1, sélectionnés parmi le groupe suivant :

- 1-(2-éthoxyéthyl)-2-{4-[4-(pyrazol-1-yl)butyl]homopipérazin-1-yl-méthyl}benzimidazole.
- 1-(2-éthoxyéthyl)-2-{4-[4-(pyrrol-1-yl)butyl)homopipérazin-1-yl-méthyl}benzimidazole.
- 1-(2-éthoxyéthyl)-2-{4-[4-(4,5-dichloro-2-méthylimidazol-1-yl)butyl]homopipérazin-1-yl-méthyl}benzimidazole.
- 1-(2-éthoxyéthyl)-2-{4-[4-(pyrazol-1-yl)butyl)homopipérazin-1-yl}benzimidazole.
- 1-(2-éthoxyéthyl)-2-{4-[4-(4-carboxypyrazol-1-yl)butyl] homopipérazin-1-yl}benzimidazole.
- 1-(2-éthoxyéthyl)-2-{4-[4-(4-éthyloxycarbonylpyrazol-1--yl)butyl]homopipérazin-1-yl}benzimidazole.

**3.** Procédé de préparation des composés selon l'une des revendications 1 et 2, caracterisé par la mise en oeuvre d'au moins l'une des opérations suivantes:

3a.- Par réaction d'un composé de formule générale IIa

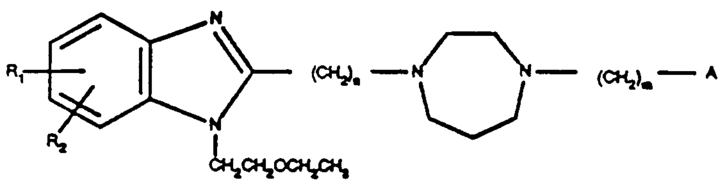

IIa

ou bien IIb

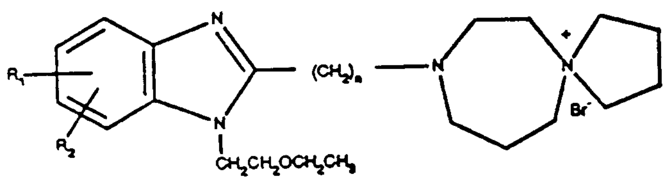

IIb

dans lesquelles $R_1$, $R_2$ représentent chacun un atome d'hydrogène, et n et m ont les significations mentionnées dans la revendication 1, et A représente un atome d'halogène, ou un bon "groupe partant" choisi parmi le tosyloxy ou le mésyloxy, avec un composé de formule générale III

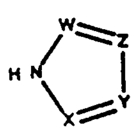

III

dans laquelle X, Y, Z et W ont les significations mentionnées dans la revendication 1.

3b.- Par réaction d'un composé de formule générale IIa, dans laquelle A représente un radical $-NH_2$, avec le 2,5--diméthoxytétrahydrofurane. 3c.- Par réaction d'un composé de formule générale IV

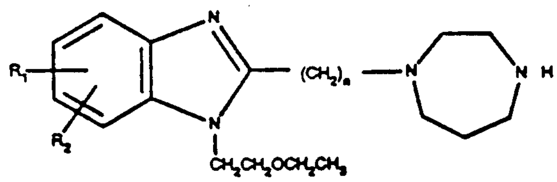

IV

dans laquelle $R_1$, $R_2$ et n ont les significations mentionnées précédemment, avec un composé de formule générale V

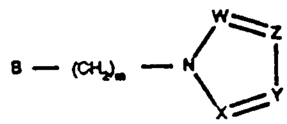

V

où X, Y, Z, W et m ont les significations mentionnées précédemment, et B représente un atome d'halogène, ou un bon "groupe partant" choisi parmi le tosyloxy ou le mésyloxy.

**4.** A titre de médicaments, les derivés de formule générale I et leurs sels thérapeutiquement acceptables, selon les revendications 1 et 2, en particulier à titre de médicaments utilisés comme antihistaminiques.

**5.** Compositions pharmaceutiques, caracterisées par le fait qu'elles contiennent, outre un support pharmaceutiquement acceptable, au moins un dérivé de formule générale I ou l'un de ses sels physiologiquement acceptables, selon l'une des revendications 1 et 2.

**6.** Utilisation des dérivés de formule générale I et leurs sels physiologiquement acceptables, selon l'une des revendications 1 et 2, pour la fabrication de médicaments destinés à la prophilaxie et au traitement de diverses maladies allergiques provoquées par l'histamine.

## Claims

**1.** Benzimidazole derivatives characterised in that they are of the general formula I, and their therapeutically acceptable salts,

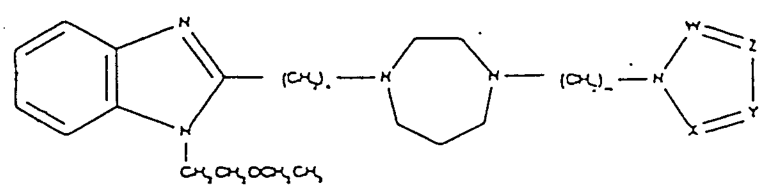

I

in which:
- n may take the values 0 or 1,
- m may take the values 2 to 4,
- X, Y, Z and W, which are identical or different, represent a nitrogen atom or a carbon atom linked to a hydrogen or to a halogen atom, or to an alkyl, carboxyl, or alkyloxycarbonyl radical.

**2.** Compounds of the general formula I according to Claim 1, which are chosen from the following group:
- 1-(2-ethoxyethyl)-2-{4-[4-(pyrazol-1-yl)butyl]homopiperazin-1-yl-methyl}benzimidazole.
- 1-(2-ethoxyethyl)-2-{4-[4-(pyrrol-1-yl)butyl]homopiperazin-1-yl-methyl}benzimidazole.
- 1-(2-ethoxyethyl)-2-{4-[4-(4,5-dichloro-2-methylimidazol-1-yl)butyl]homopiperazin-1-yl-methyl}benzimidazole.
- 1-(2-ethoxyethyl)-2-{4-[4-(pyrazol-1-yl)butyl]homopiperazin-1-yl}benzimidazole.
- 1-(2-ethoxyethyl)-2-{4-[4-(4-carboxyprazol-1-yl)-butyl]homopiperazin-1-yl}benzimidazole.

- 1-(2-ethoxyethyl)-2-{4-[4-(4-ethyloxycarbonylpyrazol-1-yl)butyl]homopiperazin-1-yl}benzimidazole.

**3.** Method of preparing the compounds according to one of Claims 1 and 2, characterised by the implementation of at least one of the following procedures:

3a. - By reacting a compound of general formula IIa

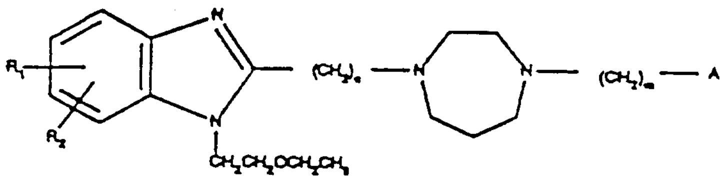

IIa

or alternatively IIb

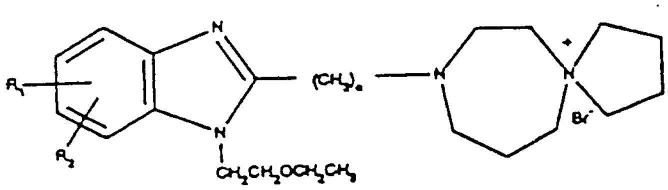

IIb

in which $R_1$, $R_2$ each represent a hydrogen atom, and n and m have the meanings given in Claim 1, and A represents a halogen atom or a good "departing group" chosen from tosyloxy or mesyloxy, with a compound of general formula III

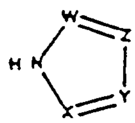

III

in which x, Y, Z and W have the meanings given in Claim 1.

3b. - By reacting a compound of general formula IIa, in which A represents a radical $-NH_2$, with 2,5-dimethoxytetrahydrofuran.

3c. - By reacting a compound of general formula IV

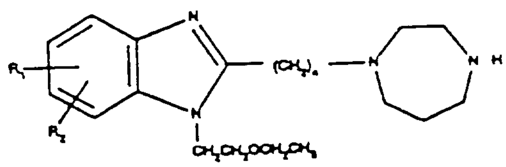

IV

in which $R_1$, $R_2$ and n have the meanings given above, with a compound of general formula V

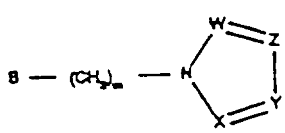

V

where X, Y, Z, W and m have the meanings given above, and B represents a halogen atom or a good "departing group" chosen from tosyloxy or mesyloxy.

4. Derivatives of general formula I and their therapeutically acceptable salts, according to Claims 1 and 2, as medicinal products, in particular as medicinal products used as antihistaminics.

5. Pharmaceutical compositions, characterised in that they contain, in addition to a pharmaceutically acceptable carrier, at least one derivative of general formula I or one of its physiologically acceptable salts, according to one of Claims 1 and 2.

6. Use of the derivatives of general formula I and their physiologically acceptable salts, according to one of Claims 1 and 2, for the manufacture of medicinal products intended for the prevention and treatment of various allergic diseases caused by histamine.

**Patentansprüche**

1. Benzimidazolderivate, dadurch gekennzeichnet, daß sie der allgemeinen Formel (I) entsprechen

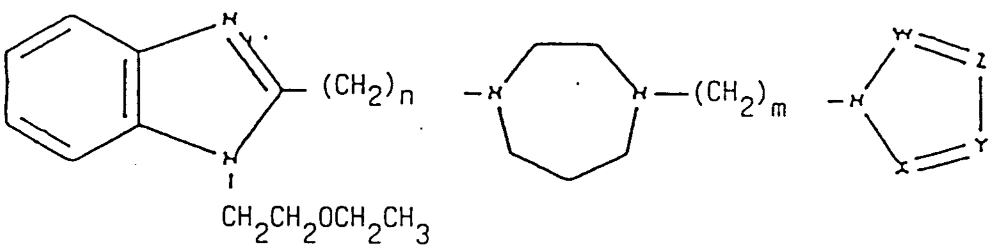

I

worin
n die Werte 0 oder 1 haben kann,
m die Werte 2 bis 4 haben kann,
X, Y, Z und W, die gleich oder verschieden sind, stehen für ein Stickstoffatom oder ein Kohlenstoffatom, das an ein Wasserstoffatom, an ein Halogenatom oder an einen Alkyl-, Carboxyl- oder Alkyloxycarbonyl-Rest gebunden ist, und ihre therapeutisch akzeptablen Salze.

2. Verbindungen der allgemeinen Formel (I) nach Anspruch 1, ausgewählt aus der folgenden Gruppe:
- 1-(2-Ethoxyethyl)-2-[4-[4-(pyrazol-1-yl)butyl]-homopiperazin-1-yl-methyl]benzimidazol,
- 1-(2-Ethoxyethyl)-2-[4-[4-(pyrrol-1-yl)butyl]-homopiperazin-1-yl-methyl]benzimidazol,
- 1-(2-Ethoxyethyl)-2-[4-[4-(4,5-dichloro-2-methylimidazol-1-yl)butyl]homopiperazin-1-yl-methyl]-benzimidazol,
- 1-(2-Ethoxyethyl)-2-[4-[4-(pyrazol-1-yl)butyl]-homopiperazin-1-yl]benzimidazol,
- 1-(2-Ethoxyethyl)-2-[4-[4-(4-carboxypyrazol-1-yl)butyl]homopiperazin-1-yl]benzimidazol,
- 1-(2-Ethoxyethyl)-2-[4-[4-(4-ethyloxycarbonylpyrazol]-1-yl)butyl]homopiperazin-1-yl]benzimidazol.

3. Verfahren zur Herstellung der Verbindungen nach einem der Ansprüche 1 und 2, dadurch gekennzeichnet, daß man mindestens eine der folgenden Reaktionen durchführt:

3a) Umsetzung einer Verbindung der allgemeinen Formel (IIa) oder (IIb)

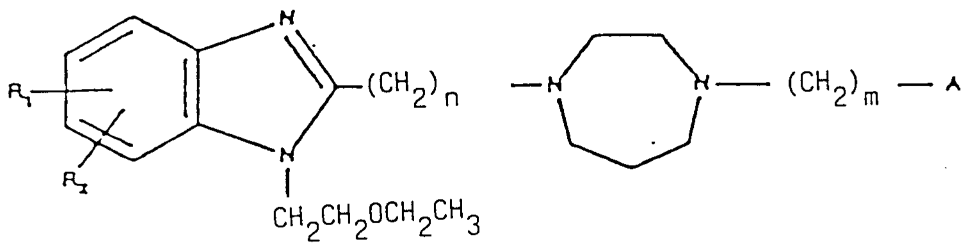

IIa

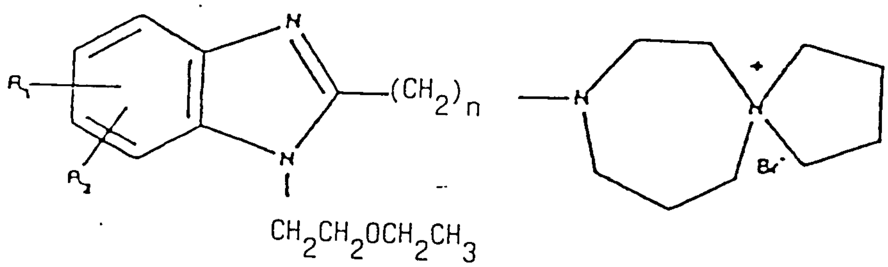

IIb

worin $R_1$ und $R_2$ jeweils ein Wasserstoffatom darstellen und n und m die in Anspruch 1 angegebenen Bedeutungen haben und A ein Halogenatom oder eine leicht "austretende(abspaltbare) Gruppe", ausgewählt aus Tosyloxy oder Mesyloxy, darstellt,
mit einer Verbindung der allgemeinen Formel (III)

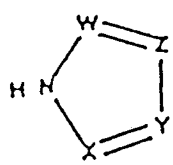

III

worin X, Y, Z und W die in Anspruch 1 angegebenen Bedeutungen haben;
3b) Umsetzung einer Verbindung der allgemeinen Formel (IIa), in der A für einen $-NH_2$-Rest steht, mit 2,5-Dimethoxytetrahydrofuran;
3c) Umsetzung einer Verbindung der allgemeinen Formel (IV)

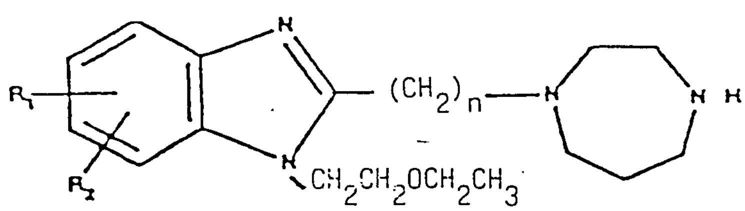

IV

worin $R_1$, $R_2$ und n die oben angegebenen Bedeutungen haben, mit einer Verbindung der allgemeinen Formel (V)

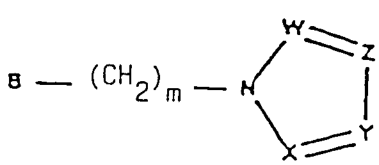

V

worin X, Y, Z, W und m die oben angegebenen Bedeutungen haben und B ein Halogenatom oder eine leicht "austretende(abspaltbare) Gruppe", ausgewählt aus Tosyloxy oder Mesyloxy, darstellt.

4. Derivate der allgemeinen Formel (I) und ihre therapeutisch akzeptablen Salze nach den Ansprüchen 1 und 2 insbesondere als Arzneimittel, die als Antihistaminika verwendet werden.

5. Pharmazeutische Zusammensetzungen, dadurch gekennzeichnet, daß sie außer einem pharmazeutisch akzeptablen Träger mindestens ein Derivat der allgemeinen Formel (I) oder eines seiner physiologisch akzeptablen Salze nach einem der Ansprüche 1 und 2 enthalten.

6. Verwendung der Derivate der allgemeinen Formel (I) und ihrer physiologisch akzeptablen Salze nach einem der Ansprüche 1 und 2 zur Herstellung von Arzneimitteln für die Prophylaxe und für die Behandlung verschiedener allergischer Erkrankungen, die durch Histamin hervorgerufen werden.